# EUROPÄISCHE PATENTSCHRIFT

(11) **EP 2 579 825 B1**
(45) Veröffentlichungstag und Bekanntmachung des Hinweises auf die Patenterteilung: **12.08.2015**
(21) Anmeldenummer: 11837278.8
(22) Anmeldetag: 03.06.2011
(51) Int. Cl.: A61F 5/56

(54) **MUNDEINSATZ**
MOUTHPIECE
INSERT DE BOUCHE

(30) Priorität: 09.06.2010 DE 102010023256
(43) Veröffentlichungstag der Anmeldung: 17.04.2013
(73) Patentinhaber: Schmitt-Bylandt, Jürgen, 63571 Gelnhausen-Haitz (DE)
(72) Erfinder: Schmitt-Bylandt, Jürgen, 63571 Gelnhausen-Haitz (DE)
(74) Vertreter: Wolf, Michael
(86) Internationale Anmeldenummer: PCT/DE2011/001229
(87) Internationale Veröffentlichungsnummer: WO 2012/062240

(56) Entgegenhaltungen:
- DE-U1- 29 509 294
- US-A- 5 879 155

## Beschreibung

Die Erfindung betrifft einen Mundeinsatz gemäß dem Oberbegriff des Patentanspruchs 1.

Ein Mundeinsatz der eingangs genannten Art ist aus dem Dokument US 5,879,155 A bekannt.

Weiterhin ist ein Mundeinsatz nach der DE 295 09 294 U1 bekannt. Dieser besteht aus einem drahtartigen Element (dort Verbindungsglied genannt), an dem zwei Aufbeißelemente (dort Kauteil genannt) befestigt sind. Das Verbindungsglied ist dabei typischer Weise aus Edelstahl oder Titan gebildet. Die Kauteile bestehen aus Kunststoff. Wie auch bei der nachfolgend zu erläuternden Erfindung dienen die Kauteile bzw. Aufbeißelemente dazu, jeweils zwischen menschlichen Backenzähnen von Ober- und Unterkiefer angeordnet zu werden, um die Zähne vor den Folgen des Zähneknirschens, Wangenbeißens oder dergleichen zu schützen.

Die Kauteile gemäß der Lösung nach der DE 295 09 294 U1 werden weitgehend formschlüssig auf die Backenzähne des Ober- oder Unterkiefers aufgesetzt. Das die Kauteile miteinander verbindende Verbindungsglied verläuft im so genannten Labial- und Bukkalbereich zwischen dem Zahnfleisch der Vorderzähne und der Ober- bzw. Unterlippe.

Der Erfindung liegt die Aufgabe zugrunde, einen Mundeinsatz der eingangs genannten Art zu verbessern.

Diese Aufgabe ist mit einem Mundeinsatz der eingangs genannten Art durch die im Kennzeichen des Patentanspruchs 1 aufgeführten Merkmale gelöst.

Nach der Erfindung ist also vorgesehen, dass die Aufbeißelemente jeweils an schenkelseitigen Übergängen zwischen den beiden Bügeln befestigt sind.

Mit anderen Worten sind nach der Erfindung und im Unterschied zur vorgenannten DE 295 09 294 U1 insgesamt zwei u-förmige Bügel vorgesehen. Der eine Bügel befindet sich dabei beim Tragen des Mundeinsatzes im Labial- und Bukkalbereich (auch Umschlagfalte genannt) des Unterkiefers und der andere im Labial- und Bukkalbereich des Oberkiefers. Die Bügel sind miteinander verbunden, und zwar jeweils schenkelseitig über entsprechend ausgebildete Verbindungsbereiche bzw. Übergänge, wobei weiterhin bevorzugt vorgesehen ist, dass beide Bügel und die Übergänge einstückig aus einem Draht, insbesondere einem mit Kunststoff ummantelten Nickel-Titan-Draht, hergestellt sind. Die Übergänge, die sich beim Tragen in der Nähe des Kiefergelenks (also schneidezahnabgewandt) befinden, sind bevorzugt ebenfalls u-förmig ausgebildet, d. h. abstrahiert betrachtet, besteht der Mundeinsatz aus vier u-förmigen Teilstücken, wobei zwei Teilstücke die u-förmigen Bügel bilden und die beiden anderen vorzugsweise ebenfalls u-förmigen Teilstücke die genannten Übergänge, die senkrecht zu den Bügeln verlaufen.

Insgesamt ergibt sich auf diese Weise ein sehr elastischer Mundeinsatz, der beim Tragen einerseits in den beiden Labial und Bukkalbereichen des Ober- und Unterkiefers geführt ist und an dem andererseits im Bereich der genannten Übergänge die Aufbeißelemente befestigt sind. Dabei weist dieser Mundeinsatz einen hohen Tragekomfort auf, verbunden mit dem Vorteil, dass der Mund behinderungsfrei geöffnet und geschlossen werden kann, und zwar ohne dass die Gefahr besteht, dass die Aufbeißelemente aus dem Mund herausfallen. Wird der Mund geöffnet, "hängen" die Aufbeißelemente (und zwar ohne - wie bei der DE 295 09 294 U1 - fest mit den Zähnen verbunden zu sein) gewissermaßen an den Übergängen zwischen den Backenzähnen, so dass sie automatisch wieder die richtige Position einnehmen, sobald der Mund geschlossen wird.

Die Maßgabe "drahtartig" bringt dabei zum Ausdruck, dass die Bügel, wie erläutert, vorzugsweise, aber nicht zwingend aus Metall gebildet sind. Ebenfalls vorstellbar ist der Einsatz von Bügeln aus Kunststoff, die entsprechend schmal und elastisch ausgebildet sind, um im jeweiligen Labial- und Bukkalbereich aufgenommen werden zu können.

Andere vorteilhafte Weiterbildungen des erfindungsgemäßen Mundeinsatzes ergeben sich aus den abhängigen Patentansprüchen.

Der Vollständigkeit halber wird noch auf folgende beiden Dokumente hingewiesen:
Aus der WO 2003/011362 A2 ist ein therapeutisches Mundstück bekannt, dass dazu dient, Mundgewebe zu wärmen oder zu kühlen. Dieses Mundstück weist keine Aufbeißelemente auf.

Aus der KR 10 2004 008 6888 A ist ein Mundstück bekannt, das mit zwei "acting agent storing units" versehen ist. Diese beiden Einheiten zur Aufnahme eines Wirkstoffs (zum Beispiel zur Behandlung von Mundgeruch) entsprechen somit schon mit Blick auf ihre Anordnung am Trägerelement (diese ist offensichtlich bewußt so gewählt, dass der Benutzer nicht darauf beißen kann) nicht den erfindungsgemäßen Aufbeißelementen.

Der erfindungsgemäße Mundeinsatz einschließlich seiner vorteilhaften Weiterbildungen gemäß der abhängigen Patentansprüche wird nachfolgend anhand der zeichnerischen Darstellung eines Ausführungsbeispiels näher erläutert.

Es zeigt schematisch
- Figur 1: perspektivisch den erfindungsgemäßen Mundeinsatz;
- Figur 2: von oben den erfindungsgemäßen Mundeinsatz; und
- Figur 3: von der Seite den erfindungsgemäßen Mundeinsatz.

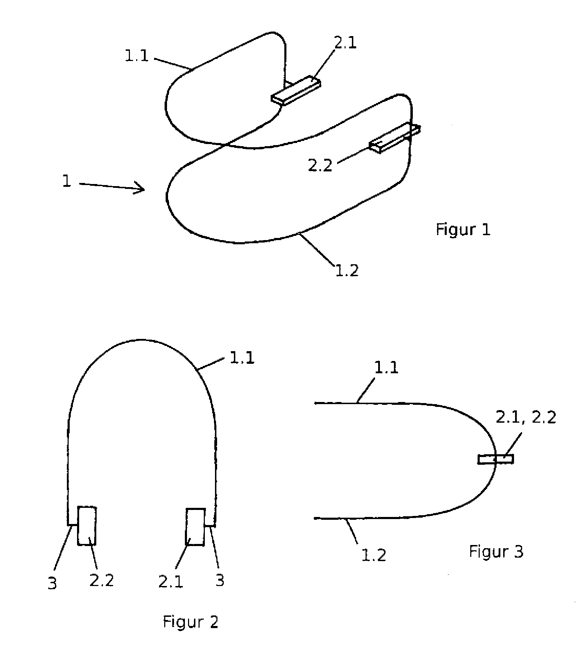

Der in den Figuren 1 bis 3 dargestellte, erfindungsgemäße Mundeinsatz besteht in bekannter Weise aus einem drahtartigen Element 1, an dem zwei Aufbeißelemente 2.1, 2.2 verdrehfest befestigt sind. Ferner ist das Element 1 aus zwei u-förmigen, jeweils beidschenklig miteinander verbundenen Bügeln 1.1, 1.2 gebildet.

Wesentlich für den erfindungsgemäßen Mundeinsatz ist nun, dass die Aufbeißelemente 2.1, 2.2 jeweils an schenkelseitigen Übergängen zwischen den beiden Bügeln 1.1, 1.2 befestigt sind.

Die u-förmigen Bügel 1.1, 1.2 sowie die Übergänge sind dabei so ausgebildet, dass sich der Mundeinsatz auch bei geöffnetem Mund noch immer mit einer gewissen Vorspannung und damit selbststabilisierend in beide Umschlagfalten legt.

Wie eingangs erläutert, könnten die u-förmigen, drahtartigen Bügel 1.1, 1.2 dabei prinzipiell zum Beispiel auch aus Kunststoff bestehen, besonders bevorzugt ist aber vorgesehen, dass die Bügel 1.1, 1.2 aus Metall, insbesondere Nickel-Titan, gebildet sind.

Um weiterhin ein Einschneiden des Metalls in die Mundschleimhaut und einen Würgereiz zu verhindern, außerdem die Last insgesamt besser zu verteilen und damit den Tragekomfort weiter zu erhöhen, ist erfindungsgemäß vorgesehen (nicht extra dargestellt), dass die metallisch ausgebildeten Bügel 1.1, 1.2 mit einer vorzugsweise aus Kunststoff gebildeten Umhüllung versehen sind.

Um den erfindungsgemäßen Mundeinsatz mit möglichst wenigen Einzelbauteilen realisieren zu können und damit auch möglichst hygienisch zu gestalten, ist weiterhin, wie in den Figuren dargestellt, vorgesehen, dass die Bügel 1.1, 1.2 und die schenkelseitigen Übergänge einstückig, also zum Beispiel aus einem (nur an einer Stelle verbundenen) Stück Nickel-Titan-Draht, ausgebildet sind.

Um scharfkantige Ecken zu vermeiden und damit den Tragekomfort weiter zu erhöhen, sind die schenkelseitigen Übergänge u-förmig ausgebildet, wobei (siehe insbesondere Figur 1) besonders bevorzugt vorgesehen ist, dass die u-förmigen Bügel 1.1, 1.2 und die u-förmig ausgebildeten, schenkelseitigen Übergänge um 90° verdreht zueinander angeordnet sind.

Weiterhin ist auch eine Ausbildung der Übergänge in der Weise denkbar (nicht dargestellt), bei der diese nicht nur u-förmig, sondern wie ein Teil einer Schraube gewendelt also loopförmig ausgebildet sind. Diese Form erhöht weiter die Flexibilität des Mundeinsatzes und führt gleichzeitig zu einer weiteren Stabilisierung der Lageposition des Einsatzes im Mund.

Zur Befestigung der Aufbeißelemente 2.1, 2.2 am drahtartigen Element 1 ist, wie insbesondere Figur 2 zeigt, an jedem schenkelseitigen Übergang ein Halteelement 3 angeordnet. Dieses ist ebenfalls vorzugsweise drahtartig und einstückig mit dem schenkelseitigen Übergang verbunden ausgebildet. Insbesondere ist vorgesehen (nicht extra dargestellt), das Halteelement 3 aus dem Draht des schenkelseitigen Übergangs mit auszuformen, so dass die Bügel, die Übergänge und die Halteelemente insgesamt nur aus einem einzigen Drahtstück gebildet sind, das an nur einer einzigen Stelle zusammengefügt, vorzugsweise thermisch gefügt, insbesondere geschweißt, werden muss.

Bezüglich der Aufbeißelemente 2.1, 2.2 ist vorgesehen, dass diese (siehe Figur 1 und 2) in Bezug auf die u-förmigen Bügel 1.1, 1.2 jeweils ins Innere des Bügel weisend angeordnet und wahlweise aus Kunststoff oder Gummi gebildet sind. Neben der rein abstützenden bzw. kiefergelenksentlastenden Funktion ist schließlich vorteilhaft vorgesehen, die Aufbeißelemente 2.1, 2.2 zur Zahnbehandlung zum Beispiel als fluoridgetränkte, schwamm- bzw. depotartige, eventuell sich langsam auflösende Medikamententräger auszubilden.

### Bezugszeichenliste

- 1: drahtartiges Element
- 1.1: u-förmiger Bügel
- 1.2: u-förmiger Bügel
- 2.1: Aufbeißelement
- 2.2: Aufbeißelement
- 3: Halteelement

## Patentansprüche

1. Mundeinsatz, umfassend ein drahtartiges Element (1), an dem zwei Aufbeißelemente (2.1, 2.2) befestigt sind, wobei das Element (1) aus zwei u-förmigen, jeweils beidschenklig miteinander verbundenen Bügeln (1.1, 1.2) gebildet ist,
**dadurch gekennzeichnet,**
**dass** die Aufbeißelemente (2.1, 2.2) jeweils an schenkelseitigen Übergängen zwischen den beiden Bügeln (1.1, 1.2) befestigt sind.

2. Mundeinsatz nach Anspruch 1,
**dadurch gekennzeichnet,**
**dass** die Bügel (1.1, 1.2) aus Metall, insbesondere Nickel-Titan, gebildet sind.

3. Mundeinsatz nach Anspruch 2,
**dadurch gekennzeichnet,**
**dass** die metallisch ausgebildeten Bügel (1.1, 1.2) mit einer vorzugsweise aus Kunststoff gebildeten Umhüllung versehen sind.

4. Mundeinsatz nach einem der Ansprüche 1 bis 3,
**dadurch gekennzeichnet,**
**dass** die Bügel (1.1, 1.2) und die schenkelseitigen Übergänge einstückig ausgebildet sind.

5. Mundeinsatz nach einem der Ansprüche 1 bis 4,
**dadurch gekennzeichnet,**
**dass** die schenkelseitigen Übergänge u-förmig ausgebildet sind.

6. Mundeinsatz nach Anspruch 5,
**dadurch gekennzeichnet,**
**dass** die u-förmigen Bügel (1.1, 1.2) und die u-förmig ausgebildeten, schenkelseitigen Übergänge um 90° verdreht zueinander angeordnet sind.

7. Mundeinsatz nach einem der Ansprüche 1 bis 6,
**dadurch gekennzeichnet,**
**dass** an jedem schenkelseitigen Übergang ein Halteelement (3) für ein Aufbeißelement (2.1, 2.2) angeordnet ist.

8. Mundeinsatz nach einem der Ansprüche 1 bis 7,
**dadurch gekennzeichnet,**
**dass** die Aufbeißelemente (2.1, 2.2) in Bezug auf die u-förmigen Bügel (1.1, 1.2) jeweils ins Innere des Bügel weisend angeordnet sind.

9. Mundeinsatz nach einem der Ansprüche 1 bis 8,
**dadurch gekennzeichnet,**
**dass** die Aufbeißelemente (2.1, 2.2) wahlweise aus Kunststoff oder Gummi gebildet sind.

10. Mundeinsatz nach einem der Ansprüche 1 bis 9,
**dadurch gekennzeichnet,**
**dass** die Aufbeißelemente (2.1, 2.2) als Medikamententräger ausgebildet sind.

## Claims

1. A mouth insert, comprising a wire-type element (1), to which two bite elements (2.1, 2.2) are attached, wherein the element (1) is formed from two U-shaped straps (1.1, 1.2), each connected to the other at both legs,
**characterized in that**
the bite elements (2.1, 2.2) are each attached to transitions on the leg side between the two straps (1.1, 1.2).

2. The mouth insert according to Claim 1,
**characterized in that**
the straps (1.1, 1.2) are made of metal, in particular nickel-titanium.

3. The mouth insert according to Claim 2,
**characterized in that**
the straps (1.1, 1.2) made of metal are provided with a sheathing, preferably made of plastic.

4. The mouth insert according to any one of Claims 1 to 3,
**characterized in that**
the straps (1.1, 1.2) and the transition on the leg side are designed in one piece.

5. The mouth insert according to any one of Claims 1 to 4,
**characterized in that**
the leg-side transitions are designed to be U-shaped.

6. The mouth insert according to Claim 5,
**characterized in that**
the U-shaped straps (1.1, 1.2) and the U-shaped transitions on the leg side are arranged so that they are rotated 90° to one another.

7. The mouth insert according to any one of Claims 1 to 6,
**characterized in that**
a holding element (3) for a bite element (2.1, 2.2) is arranged on each transition on the leg side.

8. The mouth insert according to any one of Claims 1 to 7,
**characterized in that**
the bite elements (2.1, 2.2) are arranged with each facing into the interior of the strap with respect to the U-shaped straps (1.1, 1.2).

9. The mouth insert according to any one of Claims 1 to 8,
**characterized in that**
the bite elements (2.1, 2.2) are optionally formed from plastic or rubber.

10. The mouth insert according to any one of Claims 1 to 9,
**characterized in that**
the bite elements (2.1, 2.2) are designed as medication carriers.

## Revendications

1. Insert buccal, comprenant un élément de type fil (1) sur lequel deux éléments à mordre (2.1, 2.2) sont fixés, sachant que l'élément (1) est formé par deux étriers en U, reliés entre eux, ayant chacun deux branches (1.1, 1.2)
**caractérisé en ce que**
les éléments à mordre (2.1, 2.2) sont respectivement fixés sur des jonctions côté branche entre les deux étriers (1.1, 1.2).

2. Insert buccal selon la revendication 1,
**caractérisé en ce que**
les étriers (1.1, 1.2) sont fabriqués en métal, en particulier en nickel-titane.

3. Insert buccal selon la revendication 2,
**caractérisé e n ce que**
les étriers (1.1, 1.2) fabriqués en métal sont dotés d'une gaine de préférence en plastique.

4. Insert buccal selon l'une des revendications 1 à 3,
**caractérisé en ce que**
les étriers (1.1, 1.2) et les jonctions côté branche sont conçus d'un seul tenant.

5. Insert buccal selon l'une des revendications 1 à 4,
**caractérisé en ce que**
les jonctions côté branche sont en U.

6. Insert buccal selon la revendication 5,
**caractérisé en ce que**
les étriers en U (1.1, 1.2) et les jonctions côté branche en U sont disposés en étant tournés de 90° l'un par rapport à l'autre.

7. Insert buccal selon l'une des revendications 1 à 6,
**caractérisé en ce**
**qu'**un élément de fixation (3) pour un élément à mordre (2.1, 2.2) est disposé sur chaque jonction côté branche.

8. Insert buccal selon l'une des revendications 1 à 7,
**caractérisé en ce que**
les éléments à mordre (2.1, 2.2) sont disposés respectivement orientés vers l'intérieur de l'étrier par rapport aux étriers en U (1.1, 1.2).

9. Insert buccal selon l'une des revendications 1 à 8,
**caractérisé en ce que**
les éléments à mordre (2.1, 2.2) sont fabriqués au choix en plastique ou en caoutchouc.

10. Insert buccal selon l'une des revendications 1 à 9,
**caractérisé en ce que**
les éléments à mordre (2.1, 2.2) sont conçus en tant que supports de médicament.
